# EUROPÄISCHE PATENTSCHRIFT

(11) **EP 1 694 433 B1**
(45) Veröffentlichungstag und Bekanntmachung des Hinweises auf die Patenterteilung: **09.12.2009**
(21) Anmeldenummer: 04791274.6
(22) Anmeldetag: 21.10.2004
(51) Int. Cl.: B01J 23/02, C07C 1/24, B01J 21/06

(54) **KATALYSATOR UND VERFAHREN ZUR HERSTELLUNG VON 1-OLEFINEN AUS 2-HYDROXYALKANEN**
CATALYST AND METHOD FOR THE PRODUCTION OF 1-OLEFINS FROM 2-HYDROXYALKANES
CATALYSEUR ET PROCEDE DE PREPARATION DE 1-OLEFINES A PARTIR DE 2-HYDROXYALCANES

(30) Priorität: 18.12.2003 DE 10359628
(43) Veröffentlichungstag der Anmeldung: 30.08.2006
(73) Patentinhaber: Evonik Oxeno GmbH, 45772 Marl (DE)
(72) Erfinder: KAIZIK, Alfred, 45772 Marl (DE); MASCHMEYER, Dietrich, 45657 Recklinghausen (DE); WIESE, Klaus-Diether, 45721 Haltern am See (DE); BÜSCHKEN, Wilfried, 45721 Haltern am See (DE); GAUDSCHUN, Kurt-Alfred, 45665 Recklinghausen (DE)
(74) Vertreter: Hirsch, Hans-Ludwig
(86) Internationale Anmeldenummer: PCT/EP2004/052607
(87) Internationale Veröffentlichungsnummer: WO 2005/058485

(56) Entgegenhaltungen:
- EP-A- 0 219 609
- US-A- 4 681 979
- US-A- 5 210 363

## Beschreibung

Die vorliegende Erfindung betrifft ein Verfahren zur Herstellung von 1-Olefinen aus 2-Hydroxyalkanen durch katalytische Wasserabspaltung unter nicht isomerisierenden Bedingungen sowie einen dafür besonders gut geeigneten Katalysator.

Olefine zählen wegen ihrer Reaktivität zu den wichtigsten Synthesebausteinen der organischen Chemie. Sie sind Vorstufen für eine Vielzahl von Verbindungen, wie beispielsweise von Aldehyden, Ketonen, Alkoholen, Carbonsäuren und Halogenverbindungen. In großen Mengen werden sie zur Herstellung von Homo- oder Cooligomeren und Homo- und Copolymeren verwendet, wie beispielweise Polyethylen oder Polypropylen.

Ethylen und Propylen werden durch Steamcracking oder durch katalytische Spaltung von Kohlenwasserstoffen weltweit in großen Mengen hergestellt. Dabei fallen beträchtliche Mengen an C₄-Olefinen (Isobuten, 1-Buten und 2-Butene) und in geringerem Umfang C₅-Olefine an. Höhere 1-Olefine werden meistens durch Aufbaureaktionen erzeugt.

Ethylen kann mit Hilfe von Ziegler-Katalysatoren oligomerisiert werden, wobei ein Gemisch von unverzweigten 1-Olefinen mit gerader C-Zahl anfällt.

Nach einer Variante des von Shell entwickelten SHOP-Prozesses (Cornils u. Herrmann, Applied Homogeneous Catalysis with Organometallic Compounds, Bd. 1: Applications, S. 251-256, Weinheim: VCH Verlagsges. 1996) können aus Ethylen unverzweigte 1-Olefine mit gerader und ungerader Kohlenstoffatom-Zahl hergestellt werden. Dieses Verfahren umfasst drei Reaktionsschritte, nämlich Ethylenoligomerisierung, Isomerisierung, d. h. Verschiebung der Doppelbindungen, und Kreuzmetathese des Olefingemisches mit innenständigen Doppelbindungen mit Ethylen.

Durch Dehydrierung von geradkettigen Paraffinen, beispielsweise durch Chlorierung und anschließende Dehydrochlorierung, werden Olefine mit überwiegend innenständigen Doppelbindungen erhalten, die durch Kreuzmetathese zu 1-Olefinen umgesetzt werden können. Die oben genannten Verfahren haben den gemeinsamen Nachteil, dass jeweils eine Vielzahl an 1-Olefinen erzeugt wird

Geradkettige 1-Olefine mit gerader Anzahl an Kohlenstoffatomen können z.B. aus Fettalkoholen durch Wasserabspaltung gewonnen werden. Nachteilig daran ist der hohe Preis der Einsatzstoffe und dass im Wesentlichen nur Fettalkohole mit 12 bis 18 C-Atomen in ausreichendem Maße zur Verfügung stehen.

Eine weitere Möglichkeit zur Herstellung von 1-Olefinen besteht darin, Wasser aus technisch einfach herzustellenden und damit preiswerten 2-Hydroxyalkanen und deren Mischungen abzuspalten. Die Wasserabspaltung ist eine β-Eliminierung, d. h. die Atome des abzuspaltenden Wassers sind an zwei benachbarten Kohlenstoffatomen gebunden. Bei der Wasserabspaltung aus 2-Hydroxyalkanen bilden sich 1-Olefine (Hofmann-Produkt) und/oder 2-Olefine (Saitzew-Produkte) als Primärprodukt(e). Die 2-Olefine sind thermodynamisch stabiler als das 1-Olefin, noch stabiler sind die Olefine mit weiter innenständigen Doppelbindungen. Unter isomerisierenden Bedingungen kann ein 1-Olefin bis zur Einstellung des thermodynamischen Gleichgewichts in Olefine mit innenständigen Doppelbindungen umgesetzt werden. Bei der Herstellung eines 1-Olefins ist die Bildung von Olefinen mit innenständigen Doppelbindungen aus zwei Gründen nachteilig, nämlich wegen des Ausbeuteverlustes und des Aufwands für die Abtrennung des 1-Olefins, da die Siedepunkte der isomeren Olefine nahe beieinander liegen.

Die Forderung für ein technisches Verfahren zur Herstellung von 1-Olefmen aus 2-Hydroxyalkanen oder aus deren Gemischen ist daher eine selektive kinetisch kontrollierte Wasserabspaltung (Ausbildung der Kohlenstoff-Doppelbindung zwischen dem ersten und zweiten Kohlenstoffatom) zum Zielprodukt unter Vermeidung einer anschließenden Isomerisierung.

Die Wasserabspaltung aus 2-Hydroxyalkanen wird wegen der einfachen Abtrennung der Reaktionsprodukte aus dem Reaktionsgemisch meistens an festen Katalysatoren im Temperaturbereich von 200 bis 500 °C in der Gas- oder Gas/Flüssig-Mischphase durchgeführt. Als Katalysatoren werden Oxide der Erdalkalimetalle, des Aluminiums, Indiums, Galliums, des Siliziums, Scandiums, Yttriums, Lanthans, Titans, Zirkoniums, Thoriums sowie der Seltenen Erden verwendet. Es werden auch Mischoxide und Kombinationen einiger der obigen Oxide eingesetzt. Bei einigen Katalysatoren werden durch Zugabe von Alkalioxiden eine bestimmte Acidität eingestellt.

Aus der wissenschaftlichen Fachliteratur sind für die Wassereliminierung aus 2-Hydroxyalkanen beispielsweise folgende Katalysatoren bekannt:
NiO/Al₂O₃; CuO/Al₂O₃; Al₂O₃ (J. Mol. Catal. A. Chem. (1997), 121 (2-3), S. 157-159);
ZrO₂; sulfatisiertes ZrO₂ (J. Mol. Cat. A. Chem (1997), 118 (1), S. 88-89);
Al₂O₃; Co₂O₃; ThO₂; In₂O₃ (J. Catal. (1988), 110 (2), S. 416-418);
HfO₂/ZrO₂ ( J. Phys. Chem. (1980), 84 (1), 55-56);
Al₂O₃/Na₂O; Th02 (J. Catal. (1981), 68 (2), S. 383-387);
ThO₂ ( J. Org. Chem. (1967), 32 (11), 3386-3389);
La₂O₃ (Z. Phys. Chem.(1985), 144, S. 157-163);
Ga₂O₃ ( J. Org. Chem. (1979), , 44 (13), S. 2142-2145);
ThO₂; Al₂O₃ (J. Org. Chem. (1972), 37 (8), S. 1240-1244);

An den oben genannten Katalysatoren entstehen 1-Olefine aus 2-Hydroxyalkanen in Selektivitäten von unter 90 %. Dabei entstehen, abhängig vom Katalysator im unterschiedlichen Ausmaß, auch nicht olefinische Produkte, wie beispielsweise Ether und Ketone.

Aus der Patentliteratur sind ebenfalls einige Verfahren zur Herstellung von 1-Olefinen aus sekundären 2-Alkoholen bekannt.

In EP 0 150 832 wird als Katalysator zur Eliminierung von Wasser aus sekundären 2-Alkoholen hochreines ZrO₂, das weniger als 0,3 Massen-% SiO₂ oder TiO₂ enthält, verwendet. Die Selektivität der 1-Olefmbildung an diesem Katalysator beträgt bei einem Umsatz von 90 % maximal 91 %.

Für den gleichen Zweck wird in EP 0 222 356 ein ZrO₂-Katalysator eingesetzt, dessen Acidität durch Zusatz von Alkalioxid oder Erdalkalioxid modifiziert ist. Mit Hilfe dieser Katalysatoren wird 4-Methyl-2-pentanol in einer Selektivität von 92 - 94 % zu 4-Methylpent-1-en umgesetzt. Als Nebenprodukte entstehen innenständige Olefine in einer Selektivität von 2 - 4 % und Ketone (durch Dehydrierung) in einer Selektivität von 2 - 5%.

In US-A-4 681 979, US-A-5 210 363 und EP-A-0 219 609 werden Katalysatoren für den gleichen Zweck eingesetzt, die etwa aus Zirkoniumdioxid und Alkalioxid, Yttriumoxid und Zirkoniumdioxid oder Yttriumoxid und Alkali- oder Erdalkalioxid bestehen können.

Bei einigen Verwendungszwecken, beispielsweise bei Verwendung als Comonomer, ist es notwendig, dass das entsprechende Olefin in hoher Reinheit, d. h. unter anderem mit einem geringen Gehalt an Olefinen mit innenständiger Doppelbindung, vorliegt. Die Trennung von 1-Olefin von seinen Isomeren mit innenständigen Doppelbindung ist häufig wegen der geringen Differenz der Siedepunkte mit vertretbaren Aufwand nicht durchführbar.

Da bei den bekannten Verfahren zur Dehydratisierung von 2-Alkanolen das Verhältnis zwischen gebildetem 1-Olefin und der Summe der Isomeren mit innenständiger Doppelbindung zu klein ist, bestand die Aufgabe der vorliegenden Erfindung darin, ein Verfahren zu entwickeln, das bei der Wasserabspaltung aus 2-Hydroxyalkanen und 2-Hydroxyalkane aufweisenden Gemischen neben hoher Selektivität für die Bildung von 1-Olefinen ein hohes Verhältnis von 1-Olefinen zur Summe ihrer Olefinisomeren liefert. Dies bedeutet, dass die gebildeten 1-Olefine unter Reaktionsbedingungen kaum unter Verschiebung der Doppelbindung zu innenständigen Olefinen isomerisieren dürfen.

Überraschenderweise wurde gefunden, dass durch Einsatz eines Katalysators, der formal aus Zirkoniumdioxid (ZrO₂) und Yttriumoxid (Y₂O₃) und Alkalioxid und/oder Erdalkalioxid besteht, bei der Dehydratisierung von sekundären 2-Alkoholen unter nicht isomerisierenden Bedingungen (also Einsatz eines Katalysators mit einer Zusammensetzung gemäß der vorliegenden Erfindung) Olefine in einer Selektivität von bis zu 99 % gebildet werden können und dabei 1-Olefine in einer Selektivität von bis zu 98,5 % erhalten werden.

Gegenstand der vorliegenden Erfindung ist deshalb ein Katalysator, der formal Zirkoniumdioxid (ZrO₂), Yttriumoxid (Y₂O₃) und zumindest ein Oxid, ausgewählt aus Alkali-oder Erdalkalioxid, aufweist, und bei dem der Anteil an Zirkoniumdioxid (ZrO₂) von 80 bis 99 Massenteile, der Massenanteil an Yttriumoxid (Y₂O₃) von 0,5 bis 10 Massenteile und der Anteil an Erdalkali- und/oder Alkalioxid von 0,1 bis 3 Massenteile beträgt.

Ebenfalls ist Gegenstand der vorliegenden Erfindung ein Verfahren zur Herstellung von 1-Olefinen durch katalytische Dehydratisierung (Wasserabspaltung) von Alkoholen bei einer Temperatur von 200 bis 450 °C, welches dadurch gekennzeichnet ist, dass als Katalysator ein erfindungsgemäßer Katalysator eingesetzt wird und als Alkohol zumindest ein sekundärer 2-Alkohol eingesetzt wird.

Das Verfahren unter Verwendung des erfindungsgemäßen Katalysators hat den Vorteil, dass im aus der Dehydratisierung erhaltenen Reaktionsgemisch der Anteil an Nebenprodukten, die nicht in die Dehydratisierungsstufe oder eine vorgelagerte Stufe zurückgeführt werden können, wie beispielsweise vom Edukt abgeleitete Ether, gering ist. Die Olefinfraktion kann zu 96 bis 98,5 % aus 1-Olefin bestehen, sodass die wirtschaftliche Gewinnung von reinem 1-Olefin möglich ist. Durch das erfindungsgemäße Verfahren können bei der Dehydratisierung von sekundären 2-Alkanolen (2-Hydroxyalkanen) oder von deren Gemischen Olefine in einer Selektivität bis zu 99 % gebildet werden und 1-Olefine in einer Selektivität von 95 bis 98,5 % entstehen, wenn die durch Dehydrierung entstandenen Nebenprodukte, wie z.B. Ketone nach Hydrierung zum Ausgangsalkohol wieder in die erfindungsgemäße Dehydratisierung zurückgeführt werden.

Der erfindungsgemäße Katalysator sowie das erfindungsgemäße Verfahren werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können.

Der erfindungsgemäße Katalysator, der formal Zirkoniumdioxid (ZrO₂), Yttriumoxid (Y₂O₃) und zumindest ein Oxid, ausgewählt aus Alkali- oder Erdalkalioxid, aufweist, zeichnet sich dadurch aus, dass er einen Anteil an Zirkoniumdioxid (ZrO₂) von 80 bis 99 Massenteile, der Massenanteil an Yttriumoxid (Y₂O₃) von 0,5 bis 10 Massenteile und der Anteil an Erdalkali- und/oder Alkalioxid von 0,1 bis 3 Massenteile aufweist bzw. aus diesen Bestandteilen besteht.

Vorzugsweise weist der Katalysator einen Anteil an Zirkoniumdioxid (ZrO₂) von 90 bis 98 Massenteile, insbesondere 93 bis 96 Massenteile, einen Massenanteil an Yttriumoxid (Y₂O₃) von 1,5 bis 8 Massenteile, insbesondere von 3,5 bis 6 Massenteile und einen Anteil an Erdalkali- und/oder Alkalioxid von 0,5 bis 2, insbesondere von 0,5 bis 1 Massenteile auf beziehungsweise besteht aus diesen Anteilen.

Der Katalysator kann ein oder mehrere Oxid(e) aus der Gruppe der Alkali- und Erdalkalimetalle aufweisen. Besonders bevorzugt weist der erfindungsgemäße Katalysator ein Alkalioxid, insbesondere ein Alkalioxid, ausgewählt aus Kaliumoxid oder Natriumoxid, auf. Der Katalysator kann z.B. die Form von Granulat, Tabletten, Zylindern, Ringen oder Strangextrudaten aufweisen. Dies ist insbesondere bei der Verwendung in der Dehydratisierung von Vorteil, da solche Formen einen relativ geringen Strömungswiderstand aufweisen.

Ein erfindungsgemäßer Katalysator kann z.B. wie nachfolgend beschrieben hergestellt werden: Bevorzugte Basis für die Herstellung des erfindungsgemäßen Katalysators kann ein Zirkonium/Yttrium-Mischoxid sein. Dieses kann z. B. durch gemeinsame Fällung von Zirkoniumoxid und Yttriumoxid bzw. deren Hydroxide und anschließende Entwässerung des Niederschlags hergestellt werden. Ein anderer, bevorzugter Weg zur Herstellung von Zirkonium/Yttrium-Mischoxid kann darin bestehen, aus einer wässrigen Lösung von Zirkonium- und Yttriumsalzen ein Yttrium dotiertes basisches Zirkoniumsulfat zu fällen, das in einem zweiten Schritt durch Umsetzung mit einer oder mehreren Basen zu einem Zirkonium/Yttriumoxid(hydroxid) umgesetzt wird. Dabei wird als Base vorzugsweise Ammoniak oder ein Ammoniakderivat der Kohlensäure (Ammoniumcarbonat, Ammoniumcarbamat, Harnstoff, Urethane) oder eine Kombination davon eingesetzt.

Die jeweils erhaltene feuchte Rohmasse wird vorzugsweise durch Trocknen bei 110 °C bis zur Gewichtskonstanz und anschließende Calcinierung bei Temperaturen bis maximal 350 °C, vorzugsweise 300 °C in ein Pulver umgewandelt, das formal aus Zirkoniumoxid (ZrO₂) und Yttriumoxid (Y₂O₃) besteht. Die Calcinierung kann an Luft durchgeführt werden. Die Calcinierungszeit beträgt vorzugsweise 3 bis 10 Stunden, besonders bevorzugt 3 bis 7 Stunden.

Aus dem Pulver werden bevorzugt Formkörper, wie beispielsweise Tabletten, Strangpresslinge oder Pellets, hergestellt.

Bei der Herstellung von Tabletten kann das obige Pulver mit Verarbeitungshilfsmittel und Bindemittel, beispielsweise Graphit und Methylcellulose, und Wasser vermischt werden. Diese Mischmasse wird in Platten gepresst, die bei 110 °C bis zur Gewichtskonstant getrocknet werden. Die Platten werden zerbrochen und durch Passieren durch ein Sieb (Mäschenweite 0,8 mm) zu einem rieselfähigen Granulat verarbeitet, das in einer Tablettiermaschine zu Tabletten, beispielsweise mit einer Dicke und einem Durchmesser von je 5 mm, gepresst werden kann.

Strangpresslinge können durch Extrudieren eines Teigs aus Zirkoniumoxid/Yttriumoxid-Pulver und Wasser unter Verwendung eines Zahnradextruders gewonnen werden.

Pellets, beispielsweise mit Durchmessern von 2 bis 5 mm, können aus einem Teig z. B. aus dem oben angegebenen Pulver aus Zirkoniumoxid/Yttriumoxid-Pulver und Wasser in einer Pelletiermaschine, beispielsweise System Eirich, Typ P02 mit einem Stemverwirbler, hergestellt werden.

Nach allen Verformvorgängen werden die Formkörper vorzugsweise bei 110 °C bis zur Gewichtskonstanz getrocknet und anschließend bei einer Temperatur von 500 bis 700 °C, vorzugsweise von 580 bis 620 °C calciniert. Die Calcinierzeiten können von 2 bis 7 Stunden, insbesondere von 3 bis 5 Stunden betragen.

Das Einbringung der Alkali- oder Erdalkalikomponente in den Katalysator kann vor oder nach der Formgebung erfolgen. Wenn die basische Komponente vor der Formbebung eingebracht wird, wird sie bevorzugt als wässrige Lösung in die zu verformende Masse eingearbeitet.

Wird die basische Komponente nach der Verformung aufgebracht, so geschieht dies durch Tränken oder Besprühen des fertigen Formkörpers mit vorzugsweise einer wässrigen Lösung der Base. Insbesondere beim Tränken kann durch Wahl eines bestimmten Verhältnis von Porenvolumen zu Lösungsvolumen gezielt die durchschnittliche Schichtdicke der Randzone, in der die basische Komponente abgeschieden wird, eingestellt werden. Danach erfolgt bevorzugt eine Trocknung bei vorzugsweise 110 °C bis zur Gewichtskonstanz und eine Calcinierung bei einer Temperatur von 300 bis 600 °C, insbesondere bei einer Temperatur von 350 bis 450 °C. Die Calcinierdauer kann von 3 bis 10 Stunden, insbesondere von 4 bis 6 Stunden betragen.

Bei dem Zusatz der Base vor der Verformung erhält man einen Katalysator, bei dem die basische Komponente bzw. deren Folgeprodukt über die gesamte Katalysatormasse gleichmäßig verteilt ist. Bei der Einbringung der basischen Komponente nach der Verformung entsteht ein Katalysator, der die basische Komponente bzw. deren Folgeprodukt hauptsächlich in den Randzonen enthält.

Der so hergestellte erfindungsgemäße Katalysator kann in verschiedenen katalytischen Reaktionen eingesetzt werden. Insbesondere eignet sich der erfindungsgemäße Katalysator zur Dehydratisierung von Alkoholen.

Das erfindungsgemäße Verfahren zur Herstellung von 1-Olefinen durch katalytische Dehydratisierung (Wasserabspaltung) von Alkoholen bei einer Temperatur von 200 bis 450 °C, zeichnet sich dadurch aus, dass als Katalysator ein Katalysator gemäß einem der Ansprüche 1 bis 5 eingesetzt wird und als Alkohol zumindest ein sekundärer 2-Alkohol eingesetzt wird. Im erfindungsgemäßen Verfahren kann/können ein 2-Hydroxyalkan, ein Gemisch isomerer 2-Hydroxyalkane oder ein Gemisch aus 2-Hydroxyalkanen mit unterschiedlicher Anzahl an Kohlenstoffatomen eingesetzt werden.
Im erfindungsgemäßen Verfahren können sekundäre 2-Alkohole mit der allgemeinen Struktur R-CH(OH)-CH₃ an den erfindungsgemäßen Katalysatoren zu den entsprechenden 1-Olefinen mit der allgemeinen Struktur R-CH=CH₂ umgesetzt werden. Die Gruppe R ist dabei vorzugsweise eine Kohlenwasserstoffgruppe mit 3 bis 25 Kohlenstoffatomen. Sie kann aliphatisch, geradkettig oder verzweigt, alicyclisch, alicyclisch-aliphatisch, aromatisch oder aromatisch-aliphatisch sein. Weiterhin können Methylengruppen dieser Reste durch Sauerstoff substituiert sein. Diese Verbindungen können beispielsweise durch Hydrierung von gesättigten oder ungesättigten 2-Ketonen hergestellt werden. Letztere können durch Aldolkondensation eines Aldehyds mit Aceton leicht gewonnen werden. Beispielsweise kann 2-Hydroxyoctan durch Hydrierung von Oct-3-en-2-on hergestellt werden, welches seinerseits durch Kondensation von Pentanal mit Aceton hergestellt werden kann.

Bevorzugt wird zumindest ein Alkohol eingesetzt, der 3 bis 27 Kohlenstoffatome, vorzugsweise 6 bis 16 Kohlenstoffatome und besonders bevorzugt 8 bis 12 Kohlenstoffatome aufweist. Ganz besonders bevorzugt wird als Alkohol 2-Hydroxyoctan eingesetzt. Ein oder mehrere Alkohol(e) kann/können als solche(r) oder im Gemisch mit weiteren Verbindungen, z.B. einem Verdünnungsmittel, eingesetzt werden. Als Verdünnungsmittel können inerte Gase oder Gasgemische, wie beispielsweise Stickstoff, Wasserstoff, Kohlenmonoxid, Kohlendioxid, Synthesegas, Methan oder Wasserdampf, oder unter Reaktionsbedingungen inerte organische Lösungsmittel, die vom Reaktionsaustrag leicht, z.B. durch Phasentrennung oder Destillation abgetrennt werden können, verwendet werden.

Die Dehydratisierung kann in der Gas- oder Flüssig/Gas-Mischphase durchgeführt werden. Im erfindungsgemäßen Verfahren wird die Dehydratisierung bevorzugt an suspendierten oder stückigen im Festbett angeordneten Katalysatoren kontinuierlich oder diskontinuierlich durchgeführt. Besonders bevorzugt wird die kontinuierliche Dehydratisierung an im Festbett angeordneten Katalysator durchgeführt.

Bei der kontinuierlichen Dehydratisierung können unterschiedliche Verfahrensvarianten gewählt werden. Das Verfahren kann adiabatisch, polytrop oder praktisch isotherm, d. h. mit einer Temperaturdifferenz von typischerweise kleiner als 10 °C, ein- oder mehrstufig durchgeführt werden. Im letzteren Falle können alle Reaktoren, vorzugsweise Rohrreaktoren, adiabatisch oder praktisch isotherm betrieben werden. Ebenfalls ist es möglich einen oder mehrere Reaktoren adiabatisch und die anderen praktisch isotherm zu betreiben. Bevorzugt wird die Dehydratisierung im geraden Durchgang betrieben. Sie kann jedoch auch unter Produktrückführung betrieben werden. Im geraden Durchgang beträgt der Umsatz an Alkohol bevorzugt von 50 bis 95 %.

Bei Betrieb im geraden Durchgang beträgt die spezifische Katalysatorbelastung WHSV (Weight-Hourly-Space-Velocity), angegeben in Gramm Edukt pro Gramm Katalysator pro Stunde, bevorzugt von 0,01 bis 15 h⁻¹, besonders bevorzugt von 0,1 bis 10 h⁻¹, ganz besonders bevorzugt von 0,5 bis 5 h⁻¹.

Die Temperatur in der Katalysatorschicht beträgt vorzugsweise von 200 bis 450 °C, insbesondere von 280 bis 380 °C. Die Dehydratisierung kann unter vermindertem Druck, unter Überdruck oder unter Normaldruck durchgeführt werden. Der Druck (absolut), unter dem die Dehydratisierung durchgeführt wird, beträgt vorzugsweise von 0,1 und 25 bar. Bevorzugt werden Drücke zwischen 0,2 und 10 bar, besonders bevorzugt zwischen 1 und 5 bar.

Um die Selektivität der 1-Olefinbildung möglichst groß zu halten, kann es vorteilhaft sein, wenn das Verfahren so durchgeführt wird, dass nur ein Teilumsatz des eingesetzten Alkohols erzielt wird Der Umsatz kann beispielsweise durch die Katalysatorbelastung (WHSV) eingestellt werden. Mit steigender Katalysatorbelastung sinkt der Umsatz.

Der Reaktionsaustrag aus der Dehydratisierung kann in eine Olefinfraktion, eine Alkohol aufweisende Fraktion und eine oder mehrere Nebenprodukte, wie beispielsweise Ether oder Carbonylverbindungen, wie z. B. 2-Ketone, aufweisende Fraktionen getrennt werden. Die Trennung kann z.B. durch Destillation erfolgen. Die nach der Trennung erhaltene Olefinfraktion weist vorzugsweise von 96 bis über 98 % 1-Olefine auf. Optional kann sie zu noch reinerem 1-Olefin aufgearbeitet werden. Die Alkohol aufweisende Fraktion, die den nicht umgesetzten Alkohol aufweist, wird vorzugsweise in die Dehydratisierung zurückgeführt. Das beispielsweise bei der Dehydratisierung von 2-Hydroxyalkan als Nebenprodukt entstandene 2-Keton (Octan-2-on) kann nach Hydrierung zum entsprechenden Alkohol wiederverwendet werden und bedingt somit keinen Stoffverlust. Die Hydrierung kann in einer gesonderten Hydriervorrichtung durchgeführt werden. Wenn jedoch die Ausgangsalkohole selbst durch Hydrierung hergestellt werden, kann es vorteilhaft sein, die als Nebenprodukte erhaltenen 2-Ketone in die der Dehydratisierung vorgelagerte Hydrierung einzuspeisen. Durch diese Abtrennung der Ketone aus dem bei der Dehydratisierung erhaltenen Gemisch, anschließende Hydrierung und Rückführung der erhaltenen Alkohole in die Dehydratisierung können bei der erfindungsgemäßen Dehydratisierung von sekundären 2-Alkanolen oder von deren Gemischen Olefine in einer Selektivität bis zu 99 % gebildet werden und 1-Olefine in einer Selektivität von 95 bis 98,5 % erhalten werden.

Mittels des erfindungsgemäßen Verfahrens sind Zusammensetzungen erhältlich, die zumindest ein 1-Olefin, vorzugsweise 1-Octen mit einem Anteil von über 90 Massen-%, bevorzugt über 95 Massen-%, besonders bevorzugt über 97 Massen-%, insbesondere über 98 Massen-% aufweisen. Als weitere Komponenten können diese Zusammensetzungen weitere Olefine ungleich 1-Olefine aufweisen. Im Fall von 1-Octen als 1-Olefin aufweisenden Zusammensetzungen können diese z. B. 2-, 3- und 4-Octene aufweisen.

Diese Zusammensetzungen können zur Herstellung von Aldehyden und/oder Alkoholen durch Hydroformylierung des in der Zusammensetzung, enthaltenen 1-Olefins verwendet werden. Insbesondere können diese zusammensetsungen zur Herstellung von Weichmacheralkoholen, insbesondere zur Herstellung von Nonanolen, wie z.B. Isononanol verwendet werden. Die nach dem erfindungsgemäßen Verfahren hergestellten Zusammensetzungen bzw. die Olefine können als Comonomere bei der Herstellung von Polyolefinen verwendet werden. Weiterhin können sie als Ausgangsstoff für weitere organische Synthesen verwendet werden.

Die folgenden Beispiele sollen das erfindungsgemäße Verfahren erläutern, ohne die Anwendungsbreite einzuschränken, die sich aus der Beschreibung und den Patentansprüchen ergibt.

### Beispiel 1: Herstellung eines basenfreien ZrO₂/Y₂O₃- Katalysators

Zu einer wässrigen Ammoniumsulfat-Lösung, hergestellt aus 100g Ammoniumsulfat ((NH₄)₂SO₄) und 3500 g Wasser, wurde bei 50 °C. innerhalb von einer Stunde unter Rühren eine Lösung, die aus 624,7 g Zirkonylchlorid-Hydrat (ZrOCl₂∗8H₂O), 32,2g Yttriumchlorid-Hydrat (YCl₃∗6H₂O) und 1800 g deionisiertem Wasser bestand, zugegeben. Durch gleichzeitige Zudosierung von 380 g einer wässrigen 20 Gew.%-igen Ammonialdösung wurde der pH-Wert der entstehenden Suspension zwischen 6,5 und 7,5 gehalten. Anschließend wurde bei der gleichen Temperatur eine weitere halbe Stunde gerührt. Bei dieser Umsetzung fiel ein Yttrium dotiertes basisches Zirkoniumsulfat aus, das mit Hilfe einer Filterfritte abgetrennt wurde. Der Filterkuchen wurde mit insgesamt 2000 g deionisiertem Wasser (VE-Wasser), zugegeben in mehreren Portionen, Chlorid-frei gewaschen (bis kein Chloridionen mehr im ablaufenden Filtrat nachweisbar waren). Der praktisch Chlorid-freie Filterkuchen wurde zu einer 85 °C warmen Lösung aus 450 g Harnstoff und 10 kg VE-Wasser unter Rühren innerhalb einer Stunde gegeben. Anschließend wurde bei der gleichen Temperatur 15 h nachgerührt. Nach Abkühlen auf Raumtemperatur (20 bis 25 °C) wurde der Feststoff abfiltriert. Der erhaltene Filterkuchen wurde in 2 kg VE-Wasser aufgeschlämmt und noch einmal filtriert. Der auf dem Filter liegende Feststoff wurde zehnmal mit jeweils einem kg VE-Wasser Sulfat-frei gewaschen (kein Sulfatnachweis mehr im ablaufenden Filtrat). Der praktisch Chlorid- und Sulfat-freie Filterkuchen wurde bei 110 °C bis zur Gewichtskonstanz getrocknet. Anschließend wurde dieser 6 h lang im Luftstrom bei 300 °C calciniert. Die Ausbeute an Yttriumoxid/Zirkoniumoxid betrug 440 g. Das Mischoxid bestand formal zu 94,95 % aus Zirkoniumoxid(ZrO₂) und zu 5,05 % aus Y₂O₃.

Das erhaltene Mischoxid (440 g) wurde mit 300 g VE-Wasser versetzt und zu einem Teig geknetet. Diese Masse wurde mit Hilfe eines Zahnradextruders zu zylindrischen Formkörpen mit einer Länge von 4-6 mm und einem Durchmesser von 1,25 mm gepresst Die Extrudate wurden bei 110 °C bis zur Gewichtskonstanz getrocknet und anschließend 5 Stunden lang bei 400 °C calciniert. Die fertigen Formkörper (Katalysator 1) hatten eine BET-Oberfläche von 45 m²/g (bestimmt gemäß dem BET-Verfahren durch N₂-Adsorption nach DIN 66131) und ein Porenvolumen von 0,65 ml/g (bestimmt gemäß Hg-Porosimetrie nach DIN 66133).

### Beispiel 2 (erfindungsgemäß): Herstellung eines ZrO₂/Y₂O₃-Katalysators mit Natriumoxid

200 g der Formkörper (Katalysator 1 aus Beispiel 1) wurden in einer Rotationstrommel (Drageetrommel) bei Raumtemperatur mit Hilfe einer Sprühdüse mit einer Lösung aus 2,6 g Natriumhydroxid und 130 g VE-Wasser imprägniert Die alkalisierten Formkörper wurden bei 110 °C bis zur Gewichtskonstanz getrocknet und anschließend 5 Stunden lang 400 °C calciniert. Die Formkörper (Katalysator 2) enthielten formal 1 Massen-% Natriumoxid (Na₂O).

### Beispiel 3 (Vergleichsbeispiel): Dehydratisierung mit dem in Beispiel 1 hergestellten Katalysator

Für die Dehydratisierung von 2-Octanol zu C₈-Olefinen in Gegenwart eines Katalysators in einem elektrisch beheizten Durchfluss-Festbettreaktor wurde als Edukt 2-Octanol der Fa. Fluka, mit einer Reinheit von >99,5 Gew.-%, eingesetzt. Bei dem Katalysator (Katalysator1) handelte es sich um ein Zirkonium-Yttrium-Mischoxid (94.95 Gew.-% ZrO₂ und 5,05 Gew.-% Y₂O₃) gemäß Beispiel 1.

Vor dem Eintritt in den Reaktor wurde das flüssige Edukt in einem vorgeschalteten Verdampfer bei 220 °C verdampft. Bei einer Reaktionstemperatur von 325 °C und einem Druck von 1 bar im Reaktor (Durchmesser 30 mm) wurden stündlich 27,5 g/h Edukt durch 37,8 g (30 ml) Katalysator in Extrudatform in der Gasphase, entsprechend einem WHSV-Wert von 0,7 h⁻¹, durchgeleitet. Das gasförmige Produkt wurde in einem Kühler abgekühlt und in flüssiger Form in einer Glasvorlage gesammelt.

Die GC-Analyse des Dehydratisierungsproduktes ist in Tabelle 1, Spalte 2, wiedergegeben. Nach den vorliegenden Ergebnissen wurde am Katalysator 1 bei 325 °C ein 2-Octanol-Umsatz von rd. 81 % erzielt. Der Produktaustrag enthält hauptsächlich C₈-Olefinisomere (in Summe 71,3 Gew.-%) davon rd. 67,2 % das Zielprodukt 1-Octen. Als Nebenprodukte der Dehydratisierung werden 2-Octanon und Dioctylether gebildet.

### Beispiel 4 (erfindungsgemäß): Dehydratisierung mit dem erfindungsgemäßen Katalysator

2-Octanol der Fa. Fluka wurde für die Dehydratisierung in einem Durchfluss-Festbettreaktor, wie in Beispiel 3 beschrieben, in Gegenwart eines mit Na₂O modifizierten ZrO₂/Y₂O₃-Mischoxides (Katalysator 2) aus dem Beispiel 2 eingesetzt.

Bei zwei Reaktionstemperaturen von 325 und 350 °C und einem Druck von 1 bar im Reaktor wurden stündlich 28 g 2-Hydroxyoctan (2-Octanol) durch 39,5 g Katalysator in der Gasphase, entsprechend einem WHSV-Wert von 0,7 h⁻¹, durchgeleitet. Wie im Beispiel 3, wurde das gasförmige Produkt in einem Kühler abgekühlt und in flüssiger Form in einer Glasvorlage gesammelt. Die GC-Analysen der Spaltungsprodukte bei 325 °C und 350 °C am erfindungsgemäßen Katalysator 2 sind in Tabelle 1, Spalte 3 und 4, wiedergegeben.

Wie man aus der Tabelle 1 entnehmen kann, wird das 2-Octanol an mit Na-modifiziertem ZrO₂/Y₂O₃-Oxid (Katalysator 2) im Vergleich zum unmodifizierten ZrO₂/Y₂O₃-Oxid (Katalysator 1) deutlich selektiver zum Wertprodukt 1-Octen bei geringerer Bildung von 3-und 4-Octenisomeren gespalten. Bei 325 °C wurde am Katalysator 2 ein 2-Octanol-Umsatz von ca. 60 % erzielt. Der Produktaustrag enthält in Summe ca. 52,1 Gew.-% C₈-Olefinisomere, davon ca. 97 % das Zielprodukt 1-Octen.

Durch eine Erhöhung der Reaktionstemperatur von 325 auf 350°C bei gleicher Katalysatorbelastung wurde der 2-Ocanol-Umsatz von 60 auf ca. 88 % gesteigert. Der Produktaustrag der 2-Octanol-Dehydratisierung bei 350°C enthält in Summe ca. 75,6 Gew.-% C₈-Olefinisomere, davon ca. 94 % das Zielprodukt 1-Octen.

Wie man aus der Tabelle 1 (Spalte 3 und 4) entnehmen kann, wird am erfindungsgemäßen Katalysator 2 das 2-Octanol mit einer hohen Selektivität zum gewünschten Wertprodukt 1-Octen dehydratisiert. Nach destillativen Abtrennung des Wertproduktes und der Nebenprodukte kann das nicht umgesetzte 2-Octanol in den Dehydratisierungsreaktor zurückgeführt werden. Das als Nebenprodukt gebildete 2-Octanon kann zu 2-Octanol hydriert werden.

**Tabelle 1: GC-Analyseergebnisse zu den Beispielen 3 und 4**

| | Beispiel 3 / Katalysator 1 | Beispiel 4 / Katalysator 2 | Beispiel 4 / Katalysator2 |
|---|---|---|---|
| Komponente in Gew.-% | T = 325 °C | T = 325 °C | T = 350 °C |
| 1-Octen | 47,97 | 50,65 | 70,95 |
| t-4-Octen | 0,35 | 0,00 | 0,00 |
| 3-Octene/c-4-Octen | 2,10 | 0,02 | 0,08 |
| t-2-Octen | 9,17 | 0,95 | 2,75 |
| c-2-Octen | 11,75 | 0,59 | 1,84 |
| 2-Octanon | 7,24 | 8,55 | 12,24 |
| 2-Octanol | 17,89 | 38,96 | 11,44 |
| Dioctylether | 3,49 | 0,25 | 0,61 |
| Rest | 0,04 | 0,03 | 0,09 |

## Patentansprüche

1. Katalysator, der formal Zirkoniumdioxid (ZrO₂), Yttriumoxid (Y₂O₃) und zumindest ein Oxid, ausgewählt aus Alkali- oder Erdalkalioxid, aufweist, bei dem der Anteil an Zirkoniumdioxid (ZrO₂) von 80 bis 99 Massenteile, der Massenanteil an Yttriumoxid (Y₂O₃) von 0,5 bis 10 Massenteile und der Anteil an Erdalkali- und/oder Alkalioxid von 0,1 bis 3 Massenteile beträgt.

2. Katalysator gemäß Anspruch 1,
**dadurch gekennzeichnet,**
**dass** der Anteil an Zirkoniumdioxid (ZrO₂) von 90 bis 98 Massenteile, der Massenanteil an Yttriumoxid (Y₂O₃) von 1,5 bis 8 Massenteile und der Anteil an Erdalkali- und/oder Alkalioxid von 0,5 bis 2 Massenteile beträgt.

3. Katalysator gemäß Anspruch 2,
**dadurch gekennzeichnet,**
**dass** der Anteil an Zirkoniumdioxid (ZrO₂) von 93 bis 96 Massenteile, der Massenanteil an Yttriumoxid (Y₂O₃) von 3,5 bis 6 Massenteile und der Anteil an Erdalkali- und/oder Alkalioxid von 0,5 bis 1 Massenteile beträgt.

4. Katalysator gemäß zumindest einem der Ansprüche 1 bis 3,
**dadurch gekennzeichnet,**
**dass** der Katalysator ein Alkalioxid aufweist, ausgewählt aus Kaliumoxid oder Natriumoxid.

5. Katalysator gemäß zumindest einem der Ansprüche 1 bis 3,
**dadurch gekennzeichnet,**
**dass** der Katalysator die Form von Granulat, Tabletten, Zylindern, Ringen oder Strangextrudaten aufweist.

6. Verfahren zur Herstellung von 1-Olefinen durch katalytische Dehydratisierung (Wasserabspaltung) von Alkoholen bei einer Temperatur von 200 bis 450 °C,
**dadurch gekennzeichnet,**
**dass** als Katalysator ein Katalysator gemäß einem der Ansprüche 1 bis 5 eingesetzt wird und als Alkohol zumindest ein sekundärer 2-Alkohol oder eine Mischung davon eingesetzt wird.

7. Verfahren nach Anspruch 6,
**dadurch gekennzeichnet,**
**dass** zumindest ein Alkohol, der 5 bis 27 Kohlenstoffatome aufweist, eingesetzt wird.

8. Verfahren nach Anspruch 7
**dadurch gekennzeichnet,**
**dass** als Alkohol 2-Hydroxyoctan eingesetzt wird.

9. Verfahren nach zumindest einem der Ansprüche 6 bis 8,
**dadurch gekennzeichnet,**
**dass** ein Gemisch eingesetzt wird, das weitere Alkohole und/oder Kohlenwasserstoffe sowie gegebenenfalls ein Verdünnungsmittel aufweist.

10. Verfahren nach zumindest einem der Ansprüche 6 bis 9,
**dadurch gekennzeichnet,**
**dass** die Dehydratisierung in der Gas- oder Flüssig/Gas-Mischphase durchgeführt wird.

11. Verfahren nach zumindest einem der Ansprüche 6 bis 10,
**dadurch gekennzeichnet,**
**dass** aus dem bei der Dehydratisierung erhaltenen Gemisch Ketone abgetrennt und hydriert werden und die erhaltenen Alkohole in die Dehydratisierung zurückgeführt werden.

## Claims

1. Catalyst which formally comprises zirconium dioxide (ZrO₂), yttrium oxide (Y₂O₃) and at least one oxide selected from among alkali metal oxides and alkaline earth metal oxides and in which the proportion of zirconium dioxide (ZrO₂) is from 80 to 99 parts by mass, the proportion by mass of yttrium oxide (Y₂O₃) is from 0.5 to 10 parts by mass and the proportion of alkaline earth metal oxide and/or alkali metal oxide is from 0.1 to 3 parts by mass.

2. Catalyst according to Claim 1, **characterized in that** the proportion of zirconium dioxide (ZrO₂) is from 90 to 98 parts by mass, the proportion by mass of yttrium oxide (Y₂O₃) is from 1.5 to 8 parts by mass and the proportion of alkaline earth metal oxide and/or alkali metal oxide is from 0.5 to 2 parts by mass.

3. The catalyst according to Claim 2, **characterized in that** the proportion of zirconium dioxide (ZrO₂) is from 93 to 96 parts by mass, the proportion by mass of yttrium oxide (Y₂O₃) is from 3.5 to 6 parts by mass and the proportion of alkaline earth metal oxide and/or alkali metal oxide is from 0.5 to 1 part by mass.

4. Catalyst according to at least one of Claims 1 to 3, **characterized in that** it comprises an alkali metal oxide selected from among potassium oxide and sodium oxide.

5. Catalyst according to at least one of Claims 1 to 3, **characterized in that** it is in the form of granules, tablets, cylinders, rings or extrudates.

6. Process for preparing 1-olefins by catalytic dehydration (elimination of water) of alcohols at a temperature from 200 to 450 °C, **characterized in that** a catalyst according to any of Claims 1 to 5 is used as catalyst and at least one secondary 2-alcohol or a mixture thereof is used as alcohol.

7. Process according to Claim 6, **characterized in that** at least one alcohol having from 5 to 27 carbon atoms is used.

8. Process according to Claim 7, **characterized in that** 2-hydroxyoctane is used as alcohol.

9. Process according to at least one of Claims 6 to 8, **characterized in that** a mixture comprising further alcohols and/or hydrocarbons and also, if desired, a diluent is used.

10. Process according to at least one of Claims 6 to 9, **characterized in that** the dehydration is carried out in the gas phase or the mixed liquid/gas phase.

11. Process according to at least one of Claims 6 to 10, **characterized in that** ketones are separated off and hydrogenated from the mixture obtained in the dehydration and the alcohols obtained are recirculated to the dehydration.

## Revendications

1. Catalyseur, qui comprend selon la formule du dioxyde de zirconium (ZrO₂), de l'oxyde d'yttrium (Y₂O₃) et au moins un oxyde choisi parmi un oxyde de métal alcalin ou alcalino-terreux, dans lequel la teneur en dioxyde de zirconium (ZrO₂) va de 80 à 99 parties en masse, la teneur massique en oxyde d'yttrium (Y₂O₃) va de 0,5 à 10 parties en masse et la teneur en oxyde de métal alcalin et/ou alcalino-terreux va de 0,1 à 3 parties en masse.

2. Catalyseur selon la revendication 1, **caractérisé en ce que** la teneur en dioxyde de zirconium (ZrO₂) va de 90 à 98 parties en masse, la teneur massique en oxyde d'yttrium (Y₂O₃) va de 1,5 à 8 parties en masse et la teneur en oxyde de métal alcalin et/ou alcalino-terreux va de 0,5 à 2 parties en masse.

3. Catalyseur selon la revendication 2, **caractérisé en ce que** la teneur en dioxyde de zirconium (ZrO₂) va de 93 à 96 parties en masse, la teneur massique en oxyde d'yttrium (Y₂O₃) va de 3,5 à 6 parties en masse et la teneur en oxyde de métal alcalin et/ou alcalino-terreux va de 0,5 à 1 partie en masse.

4. Catalyseur selon au moins l'une des revendications 1 à 3, **caractérisé en ce que** le catalyseur comporte un oxyde de métal alcalin choisi parmi l'oxyde de potassium et l'oxyde de sodium.

5. Catalyseur selon au moins l'une des revendications 1 à 3, **caractérisé en ce que** le catalyseur présente la forme de produit granulé, de comprimés, de cylindres, d'anneaux ou d'extrudats en boudins.

6. Procédé pour la production de 1-oléfines par déshydratation (élimination d'eau) catalytique d'alcools à une température de 200 à 450 °C, **caractérisé en ce qu'**on utilise comme catalyseur un catalyseur selon l'une quelconque des revendications 1 à 5 et comme alcool au moins un 2-alcool secondaire ou un mélange de tels alcools.

7. Procédé selon la revendication 6, **caractérisé en ce qu'**on utilise au moins un alcool qui comporte de 5 à 27 atomes de carbone.

8. Procédé selon la revendication 7, **caractérisé en ce qu'**on utilise comme alcool le 2-hydroxyoctane.

9. Procédé selon au moins l'une des revendications 6 à 8, **caractérisé en ce qu'**on utilise un mélange qui comporte d'autres alcools et/ou des hydrocarbures ainsi qu'éventuellement un diluant.

10. Procédé selon au moins l'une des revendications 6 à 9, **caractérisé en ce qu'**on effectue la déshydratation en phase gazeuse ou mixte gazeuse/ liquide.

11. Procédé selon au moins l'une des revendications 6 à 10, **caractérisé en ce qu'**on sépare les cétones du mélange obtenu dans la déshydratation et on les soumet à une hydrogénation, et on renvoie dans la déshydratation les alcools obtenus.
